# EUROPEAN PATENT APPLICATION

(11) **EP 2 418 596 A1**
(43) Date of publication of application: **15.02.2012**
(21) Application number: 10425273.9
(22) Date of filing: 11.08.2010
(51) Int. Cl.: G06F 19/00

(54) **A system for monitoring patients that perform a medical self-check and relative method**

(71) Applicant: Akern S.r.L., 50065 Pontassieve (FI) (IT); Marris, Jörg, 61462 Königstein (DE)
(72) Inventor: Talluri, Antonio, 50012 Bagno a Ripoli (FI) (IT); Marris, Jörg, 61462 Königstein (DE)
(74) Representative: Turini, Laura

(57) **Abstract**

The present invention refers to a system (1) for monitoring patients that perform a medical self-check. The system comprising an equipment (2) provided with:
- Data entry means (5) through which the user can enter one or more than one data in the equipment (2);
- User identification means (6) comprising a mother board (6) configured to request a univocal identification datum and verify the identification entered by the user after the said request;
- Clinical data acquisition means (3, 4, 11, 12, 13, 14) configured to acquire one or more than one user's clinical data after the positive result of the identification and;
- A mobile telephony system (8, 9);

and wherein the said system (1) further comprises at least one, preferably a plurality of diagnostic centres (20) located in different parts placed in communication with the said equipment (2) through the mobile telephony system in such a way that, in use, the equipment (2) transmits the data acquired through the said mobile telephony system (8, 9) to a diagnostic centre (20) so that the centre can monitor/file such data.

## Description

### Technical Field

The present invention refers to the technical field relative to self-check medical instruments.

In particular, the invention refers to a system and relative method that allows, following the identification of the patient, to acquire multiple clinical data of the person so as to be able to send the said data to a specific processing and monitoring centre.

### Background Art

It is known that equipments suitable for medical self-check are always more and more common in Italy. Such equipments can be used at home or can be present in public places, such as pharmacies.

Among these equipments those that can measure and check the pressure or the body weight, for example, are very common.

The same applicant has also filed a patent application in Italy with the title "A procedure and a home device for self-checking the hydration level of a person", with a publication number ITFI20050202, wherein an equipment is described that allows the patient to monitor his own correct body hydration status.

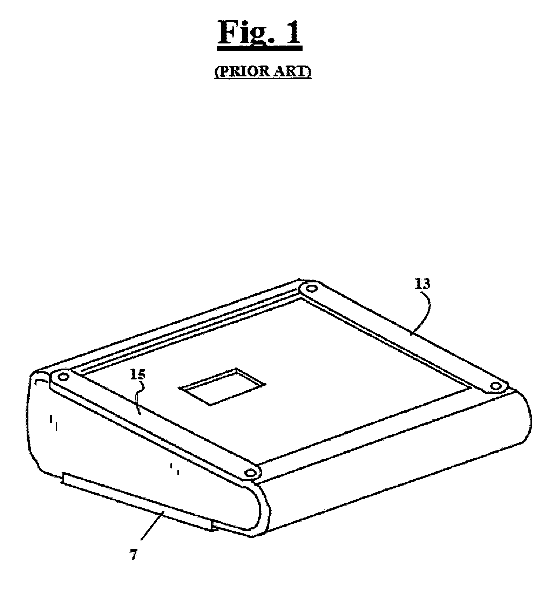

The said invention describes a compact equipment able to measure the body reactance Xc. To that aim, the equipment, shown in figure 1, is provided with a generator able to generate a reference signal of alternate tension at 50Khz that is injected in the said body section through the pair of plates (13, 9; 15; 7). The signal goes through the body from the plates A, A towards the plates B, B or rather from the fingertip of the thumb towards the fingertips of the other fingers of the hand and that lean on the said plates 13, 15. A discriminator 17 calculates the deviation of the input signal (the one injected from the plates 7, 9) with respect to the output signal (the one that arrives at the plates 13, 15) after having gone through such body part. A calculation circuit 19 calculates, on the basis of the said variation, the reactance value, showing it on the "display".

The user has been previously instructed by a specialized centre on the maximum reactance variations allowed which are representative, in accordance with well known formulas, of an equivalent variation of the specific hydration status of the person.

Considering the above, various technical inconveniences are anyway evident.

In the first place, there does not exist currently a single equipment able to acquire and process multiple data in order to provide the user with a complete case history. There only exist single machines, each one suitable for the check of a single parameter of the patient (for example the pressure check or the monitor of the said body hydration through the calculation of reactance Xc). The patient is therefore obliged to perform multiple checks in order to acquire a number of clinical data of his own interest, which is evidently a disadvantage for the person.

Moreover, it is clear that a self-check, being directed to persons not necessarily specialized in the medical field, can certainly not include complex clinical data which inevitably require medical interpretation. In that sense, self-check machines are therefore limited to monitoring a few types of parameters.

As a consequence of what has just been said, it is clear that the need to create a complete case history always requires the intervention of competent medical staff as well as specific machines. This obliges the patient to make continuous visits to specialized centres, which implies many inconveniences.

Moreover, the person is not always able to assess the own data acquired, and many times there is the risk of underestimating clinical situations that would deserve at least monitoring by specialized medical staff.

### Disclosure of invention

It is therefore the aim of the present invention to provide a patient monitoring system that solves at least in part the above-mentioned inconveniences.

In particular, it is the aim of the present invention to provide a patient monitoring system (1) which, though maintaining the peculiarity and the comfort of the self-check, allows to acquire multiple information that can be assessed in real time by specialized staff so as to create a complete case history and allows, at the same time, continuous monitoring.

It is therefore the aim of the present invention to provide a patient monitoring system (1) which, though maintaining the peculiarity of the self-check, allows an immediate specialized intervention in case the circumstances require it without leaving the result to the free interpretation of the person.

These and other aims are therefore obtained through a system (1) as per claim 1.

In particular, the said system (1) comprises an equipment (2) through which it is possible to obtain one or more than one clinical data of the patient that performs his routine self-check, for example at home or in an equipped pharmacy. In such a case, the equipment is provided with appropriate data entry-data means (5), for example a keyboard (5) or an audio/receiver system able to capture the user's voice, and through which the user can enter one or more than one data in the equipment (2).

The equipment is also provided with user identification means (6) comprising a mother board (6) configured to request a univocal user identification datum and verify the identification entered by the user after the said request.

In such a manner, the user through the keyboard (or equivalent system) enters the personal identification datum in such a way that, once the correctness of the inserted datum is verified, the continuation of the procedure can take place.

The equipment is further provided with clinical data acquisition means (3, 4, 11, 12, 13, 14) configured to acquire one or more than one user clinical data after the positive result of the identification.

The equipment (2) is also provided with a mobile telephony system (8, 9) in communication with one or more than one receiving diagnostic centres (20) located in different parts of the territory.

In such a manner, in use, the equipment (2) transmits the clinical data acquired relative to the user through the mobile telephony system (8, 9) to one of the said diagnostic centres (20) so that the centre can monitor/file such data. The clinical data can be multiple and comprising, for example, body resistance and reactance values as well as body pressure values, biometrical data in general, body weight, etc.

It is therefore clear now that all the established aims of the invention have been reached. In particular, it is now clear that, though maintaining the comfort of a home self-check, it is anyway possible to have continuous distance monitoring of the user by expert medical staff that runs the diagnostic centre, exactly as if the check was performed at a specialized centre.

Moreover, the big quantity of clinical data that can be acquired can provide a complete case history not limited exclusively to just the body hydration level.

In that sense, the medical staff can intervene on many fronts at the same time.

Advantageously, the said data entry-data means (5) can comprise a keyboard (5).

Advantageously, the identification of the user takes place through a comparison, by means of a processor (11), of an identification datum entered by the user with a reference datum of the user memorized on a memory (14, 15).

Advantageously, the clinical data acquisition means comprise two pairs of electrodes (3, 4) suitable for being grasped by the user and configured to inject an entering tension and detect an exiting tension from the user's body in such a way that the processor (11) calculates the body resistance R and reactance Xc of the person on the basis of the said difference of tension detected.

Advantageously, the said data acquisition means can further comprise one or more than one specific questions elaborated by the processor (11) and which the user answers through the data acquisition means (5), preferably through the keyboard, in such a way that the said data are acquired by the equipment.

Advantageously, a display is comprised that allows to show the data requested by the equipment to the user in such a way that the user can appropriately enter them, for example the questions the patient has to answer or the initial identification data.

Advantageously, a volatile memory (14) is further comprised in which all the data acquired are memorized ,before the sending to the diagnostic centre (20).

Advantageously, the diagnostic centres (20) are furnished with telephony system in communication with the telephony system (8, 9) of the equipment (2) and with a server provided with a software suitable for processing/filing the data received.

In such a manner, the staff of the diagnostic centre is able to establish a contact directly with the patient.

Advantageously, the server is configured to detect the body hydration status on the basis of the resistance and reactance values received.

It is also described here a method for monitoring patients that perform a medical self-check through a self-check equipment (2) and comprising the operations of:
- Entry of at least one user identification datum in the equipment (2) and subsequent identification of the user through a comparison of the datum entered by the user with a reference datum memorized in the equipment (2);
- In case of positive identification of the user, subsequent acquisition of one or more than one clinical data of the patient;
- Sending of the said data acquired through a mobile telephony system (8, 9) to a diagnostic centre (20);
- Reception of the said data by the diagnostic centre through a telephony system and subsequent processing/filing of the said data through a server in such a way that the staff can use such data when needed.

Advantageously, the entry operation of at least one identification datum is realized through a keyboard (5) of the equipment (2) through which the user enters in a processor (11) a personal identification datum, such as a password and/or User-ID.

Advantageously, the operation of user identification comprises a comparison through the processor (11) between the datum entered by the user and a user recognition datum memorized on a memory (14, 15) of the equipment.

Advantageously, the operation of data acquisition comprises only one of the said two options or a combination of the said two options:
- The detection of the patient's resistance **R** and reactance **X**c value through electrodes (3, 4) that are grasped by the user to inject an entering tension and detect an exiting tension through the user's body in such a way that the processor (11) calculates the body resistance **R** and reactance **X**c of the person on the basis of the said difference of tension detected;
- The acquisition of one or more than one clinical data of the patient through the showing by means of a display (16) of one or more than one questions elaborated by the processor (11) and to which the patient answers through the keyboard so that his answers can be acquired by the processor (11).

Advantageously, the operation of sending of the data acquired comprises the sending of an SMS containing the said data to a chosen diagnostic centre.

Advantageously, following the reception of the said data there exists the possibility of contacting the user directly by the diagnostic centre (20) through an SMS, a telephone call or a message sent through the display of the equipment.

### Brief description of drawings

Further features and advantages of the present system and relative method, according to the invention, will result clearer with the description of one embodiment that follows, made to illustrate but not to limit, with reference to the annexed drawings, wherein:
- Figure 1 shows an equipment for monitoring the body hydration level in accordance with the background art.
- Figure 2 shows a block diagram of the system 1 in accordance with the present invention.
- Figure 3 shows an axonometric view of the present equipment that highlights the grasping electrodes 3 and 4;
- Figure 4 schematizes the body resistance and reactance evaluation through the passage of current between the electrodes 3 and 4.
- Figure 5 shows a block diagram of the functioning method.

### Description of some preferred embodiments

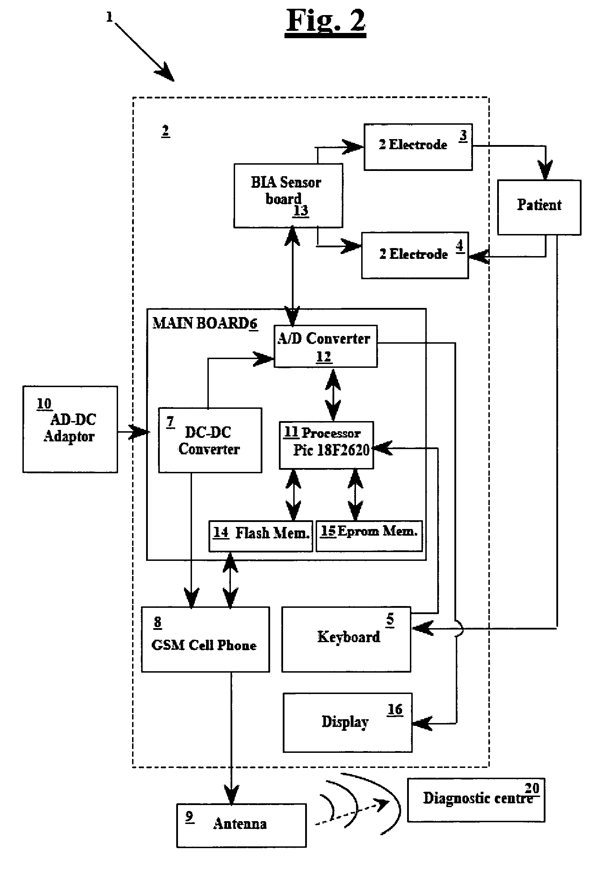

In figure 2, according to a block diagram, the general equipment of the present system 1 in accordance with the invention is schematized.

The block diagram shows the two pairs (3, 4) of electrodes with which the equipment 2 is provided in order to assess, as it is well known in the background art, the body resistance **R** and reactance **X**c values which, as better explained below in the present description, will be interpreted by specialized staff (for example, for the classic assessment of the body hydration status of the person).

The box in dotted line encloses all the essential components of such an equipment 2.

In particular, a keyboard 5 is highlighted, with which such equipment is provided and through which the patient can now interact in order to enter specific data requested. It is clear that the keyboard 5, though represented in the block diagram directly integrated to the structure of the equipment 2 itself, can be arranged externally and connected through ordinary electrical wiring. The block diagram shows with an arrow the connection between the patient and the keyboard 5 so as to exemplify schematically such machine/patient interaction. Going on in the description of the block diagram of figure 2 the connection between the keyboard 5 and the mother board 6 of the equipment 2 is highlighted, particularly a processor 11, which contains all the basic electronic elements to perform the functions described below. In particular, there is a connection between keyboard and processor 11 of the mother board, and figure 2 shows with an arrow the physical and communication connection between the keyboard and the said mother board 6 through the said processor.

The equipment 2 comprises a transformer AD-DC 10 able to provide a stable voltage of 12 V to the mother board 6 to feed the functioning of all the components. The mother board 6, in turn, comprises a further transformer DC-DC 7 which serves to simultaneously provide a first voltage of 9V, destined to some components suitable for functioning with such tension, and a second voltage of 5V destined to other components suitable for working with the said second voltage.

In particular, the transformer 7 feeds on one side a GSM mobile telephone 8 connected to an emitting/receiving antenna 9. On the opposite side the transformer 7 feeds the processor 11, preferably of the Pic 18F2620 type, through a converter A/D 12 (analogical/digital converter).

The converter A/D 12 puts in communication a sensor 13 with the processor 11 in such a way that the sensor, as per the background art described, can measure the difference of tension between the ends of the electrodes (3, 4) and the processor can receive and elaborate through the converter 12 such information in order to obtain the body resistance **R** and reactance **X**c value of the person through an appropriate software that implements specific and well known calculation formulas.

Always as shown in figure 2, two memories (14, 15) are also included. A first memory 14 is of the volatile type, while the second memory 15 is of the permanent type. The memories are connected to the processor 11 in such a way that the data processed by the processor can be memorized appropriately.

The converter A/D and the processor 11 are also connected to the display 16 for the normal showing of the data.

Last, the diagram of figure 2 shows a data collection and processing centre 20, for example a medical centre provided with an appropriate receiving system, in communication with the equipment 2 through the mobile telephone. The figure in fact schematizes an exchange of information from the antenna 9 towards the chosen centre 20 and eventually vice versa.

The data collection and processing centre 20, as it is clarified below, is naturally furnished not only with a telephony communication system in communication with the specific equipment 2, but it is also furnished with appropriate servers with a software able to process, according to well known formulas, the various data received.

It is naturally to be understood that, as clarified below in the description, the present system can comprise a plurality of the said equipments 2 placed in the territory (both home equipments and equipments arranged in public places such as pharmacies) and one or more than one diagnostic centres 20 in communication with the said equipments.

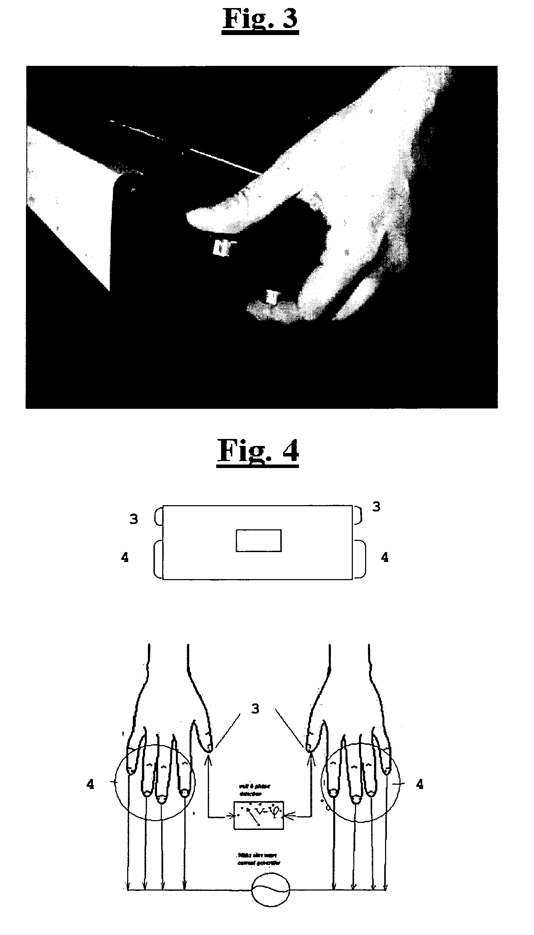

Figure 3 shows, for clarification purposes, a possible arrangement of the pairs of electrodes 3 and 4, while figure 4 shows the current flow passing through the fingertips of the patient by means of the said electrodes in such a way as to assess the body resistance and reactance of the person.

Having structurally described all the basic elements of the invention, we now pass onto a description of its functioning.

The patient that has to perform a self-check activates the equipment 2. As it was said also in the preamble of the background art, the equipment can be the equipment owned by the patient or can be found in a public place such as a pharmacy.

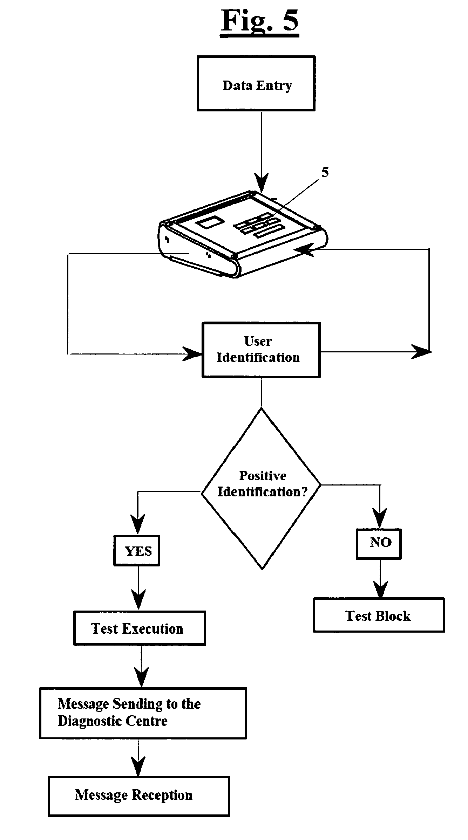

As per the flow chart of figure 5, the first step required is the univocal identification of the patient. In that sense, the equipment 2 is set, through the processor 11, to request, through the display, the entry of an identification datum. Through the keyboard 5 the patient therefore enters in the mother board 6, in particular in the processor 11, the said datum, such as a password and/or a User-ID. The processor executes the identification of the user that comprises the comparison of the identification data entered by the patient with those present in the memory, in particular in the permanent memory 15, in such a way as to approve the prosecution of the procedure. In that sense, given the lack of identification code it is not possible to continue with the check and the procedure is interrupted.

In case of positive identification, the patient can continue with the self-check procedure.

The self-check procedure comprises a calculation of the resistance **R** and reactance **X**c of the person through the injection of current in the body by means of electrodes and the processing of such data by the processor, as per figure 3 and 4.

The processor is also programmed to request the patient further information that may be useful to the specialized staff for the assessment of a general or specific case history. Therefore, the patient can, through the keyboard, enter the data that are requested to him on the display. These data can comprise, for example, the age or the body weight, as well as other specific questions to which he can answer for example through a (Yes) or (No) (for example if he has recently felt ill or which his food habits are). The questions can be similar to those that a specialist doctor would make to an own patient that visits him.

Once such data acquisition phase is completed, the data is memorized on the volatile memory 14 in such a way that the processor can control the sending of a telephonic SMS through the telephone 8 and the antenna 9. The SMS naturally contains all the information acquired which is thus sent to one or more than one diagnostic centres 20 located in the territory.

The permanent memory, apart from containing the identification of each registered patient in order to start the procedure, can also contain, for each patient, the telephone number of the centre to which he is assigned.

The centre 20 that receives such data in substantially real time is now able, through its own servers, to memorize and process them at the discretion of the staff.

For example, the chosen doctor can use the resistance and reactance values received in order to calculate and monitor the hydration level of the person. Likewise, the cardiologist could interpret other data extrapolated through the specific answers entered by the patient to assess the general cardiac status, while the nutritionist could make his assessment on the basis of data such as weight and food habits. In any case, such data is available to the specialized staff that can interpret and use them as they deem better.

The diagnostic centre is furnished with a telephony system able to communicate with the specific equipment 2 from which it has received the information.

This allows, in multiple ways, to intervene in real time in the case in which the data acquired show particularly worrying anomalies.

For example, in a particularly serious case, the specific operator can inform by telephone the patient or the public place where the equipment is found in such a way as to instruct him on the procedure to be followed and calm him down.

In less serious cases, for example, a signal can be sent by the diagnostic centre to the equipment 2 which, always through the processor, shows on the display a message addressed to the patient.

In other cases, the sending of an SMS can be configured, which indicates the result of the exam and suggests further actions such as, for example, to eventually make an appointment with a doctor.

It is therefore clear that the system just described, leaving all the advantages obtained through the self-check unaltered, allows a monitoring and processing of the data by specialized staff exactly as if the check was being performed in a specialized diagnostic centre.

Moreover, such data, thus arranged, lend themselves well to a comparison between different specialists with the purpose of having a complete case history.

## Claims

1. A system (1) for monitoring patients that perform a self-check and comprising an equipment (2) provided with:
- Data entry means (5) through which the user can enter one or more than one data in the equipment (2);
- User identification means (6) comprising a mother board (6) configured to request a univocal user identification datum and verify the identification entered by the user who made the said request;
- Clinical data acquisition means (3, 4, 11, 12, 13, 14) configured to acquire one or more than one user clinical data after the positive result of the identification and;
- A mobile telephony system (8, 9);
and wherein the said system (1) further comprises at least one, preferably a plurality of diagnostic centres (20) placed in different parts and placed in communication with the said equipment (2) through the mobile telephony system in such a way that, in use, the equipment (2) transmits the data acquired through the said mobile telephony system (8, 9) to a diagnostic centre (20) so that the centre can monitor/file such data.

2. A system (1), according to claim 1, wherein the said data entry means (5) comprise a keyboard (5).

3. A system (1), according to claim 1 or 2, wherein the said user identification takes place through a comparison, by means of a processor (11), of the datum entered by the user with a reference datum of the user memorized on a memory (14, 15).

4. A system (1), according to claim 1, wherein the said clinical data acquisition means comprise two pairs of electrodes (3, 4) suitable for being grasped by the user and configured to inject an entering tension and detect an exiting tension from the user's body in such a way that the processor (11) calculates the body resistance **R** and reactance **X**c of the person on the basis of the said difference of detected tension.

5. A system (1), according to one or more of the preceding claims, wherein the said data acquisition means further comprise one or more than one specific questions elaborated by the processor (11) and which the user answers through the said data entry means (5) in such a way that the said data are acquired by the equipment.

6. A system (1), according to one or more of the preceding claims, wherein a display is comprised that allows the display of the data requested by the equipment to the user in such a way that the user can appropriately enter them.

7. A system (1), according to one or more of the preceding claims, wherein a volatile memory is further comprised (14) into which all the data acquired are memorized before the sending to the diagnostic centre (20).

8. A system (1), according to one or more of the preceding claims, wherein the said diagnostic centres (20) are furnished with telephony systems in communication with the telephony system (8, 9) of the equipment (2) and with a server provided with a software suitable for processing/filing the data received.

9. A system (1), according to claim 8, wherein the said server is configured to detect the body hydration status on the basis of the resistance and reactance values received.

10. A method for monitoring patients that perform a medical self-check through a self-check equipment (2) and comprising the operations of:
- Entry of at least one identification datum in the said equipment (2) and subsequent identification of the user through a comparison of the datum entered by the user with a reference datum memorized in the equipment (2);
- In case of positive identification of the user, subsequent acquisition of one or more than one clinical data of the patient;
- Sending of the said acquired data through a mobile telephony system (8, 9) to a diagnostic centre (20);
- Reception of the said data by the diagnostic centre through a telephony system and subsequent processing/filing of the said data through a server in such a way that the staff can use the said data when needed.

11. A method, according to claim 10, wherein the said entry operation of at least one identification datum is realized through a keyboard (5) of the equipment (2) through which the user enters in a processor (11) a personal identification datum, such as a password and/or User-ID.

12. A method, according to claim 10, wherein the said user identification operation comprises a comparison through the processor (11) between the datum entered by the user and a user recognition datum memorized on a memory (14, 15) of the equipment.

13. A method, according to claim 10, wherein the said data acquisition operation comprises only one of the said two options or a combination of the said two options:
- The detection of the patient's resistance **R** and reactance **X**c value through electrodes (3, 4) that are grasped by the user to inject an entry tension and detect an exit tension through the user's body in such a way that the processor (11) calculates the body resistance **R** and reactance **X**c of the person on the basis of the said difference of tension detected;
- The acquisition of one or more than one clinical data of the patient through the display by means of a display (16) of one or more than one questions elaborated by the processor (11) and to which the patient answers through the keyboard so that his answers can be acquired by the processor (11).

14. A method, according to claim 10, wherein the said sending operation of the acquired data comprises the sending of an SMS containing the said data to a chosen diagnostic centre.

15. A method, according to claim 10, wherein, following the reception of the said data there exists the possibility of contacting the user directly by the diagnostic centre (20) through an SMS, a telephone call or a message sent through the display of the equipment.
